# EUROPEAN PATENT APPLICATION

(11) **EP 4 282 326 A1**
(43) Date of publication of application: **29.11.2023**
(21) Application number: 23174004.4
(22) Date of filing: 17.05.2023
(51) Int. Cl.: A61B 5/00, G16H 30/20, G16H 30/40, G16H 50/30

(54) **PROCESS FOR CODING AN IMAGE OF A SCALP ILLUMINATED WITH A FLUORESCENCE-BASED WOOD'S LIGHT**

(30) Priority: 23.05.2022 IT 202200010685
(71) Applicant: Sanders S.r.l., 20124 Milano (IT)
(72) Inventor: MANCINI, Biancamaria, Roma (IT)
(74) Representative: Rapisardi, Mariacristina

(57) **Abstract**

A process for coding an image of a scalp illuminated with a fluorescence-based Wood's light, comprising the steps of:
-extracting from the image a number of luminous spots per surface unit
-extracting from the image a dimension of the luminous spots
-extracting from the image a colour of the luminous spots
-extracting from the image a brightness of the luminous spots
-generating an alphanumeric code for coding the image made up of a first code field with a value associated with the colour of the luminous spots extracted from the image and with the dimension of the luminous spots extracted from the image, a second code field with a value associated with the brightness of the luminous spots extracted from the image, and a third code field with a value associated with the number of luminous spots per surface unit extracted from the image.

## Description

The present invention relates to a process for coding an image of a scalp illuminated with Wood's light.

### Prior art

The present invention relates in particular to the field of trichology, which deals with the physiological or paraphysiological conditions of the scalp and hair.

Thinning and the baldness are the result of a long and gradual process of degeneration of the follicle, whose causes are multifactorial. It is by now well known that the causes of thinning include alterations of the scalp, such as hyperseborrhea, reddening and flaking, often associated with inflammatory states.

Although sebum is an important element, because it contributes to the formation of a hydrolipidic film on the scalp, in the presence of an alteration of its production by the sebaceous glands, however, it can contribute to and/or accelerate hair loss.

In fact, the substances making up sebum, such as fatty acids, cholesterol, triglycerides and others, render the skin's pH too acidic and as a consequence they alter the metabolism of the scalp and hair, contributing to the agglomeration of unsightly oily flakes.

Furthermore, such problems of the scalp are often associated with itching and trichodynia, which greatly impair the person's quality of life, no longer only from an aesthetic viewpoint.

An analysis of the conditions of the scalp, and in particular of the states of oxidation of the sebum accumulated on the scalp, is normally carried out by means of:
- an objective examination of the state of health of the scalp
- a further investigation using a microcamera with a 50x magnification objective and a fluorescence-based Wood's light.

The analysis described is thus only of a qualitative type and produces solely descriptive data. For this reason, the professional's sensitivity in reporting the data detectable from the images viewed becomes very important.

Furthermore, the description of an image is expressed in qualitative terms and does not lend itself to being included in a database or within a numerical analysis and thus does not facilitate the collection of data or their systematicity and repeatability.

It is thus clear that, in this context, the experience and professionalism of the operator performing the analysis counts for a great deal and it is clear that the variability of the result is due to the operator's sensitivity and experience.

The task of the present invention is thus to provide a process for coding an image of a scalp illuminated with a fluorescence-based Wood's light which enables the described drawbacks of the prior art to be overcome.

Within the scope of this technical task, one object of the present invention is to provide a process for coding an image of a scalp illuminated with a Wood's light which enables an image of a scalp to be coded in order to represent the relevance of the accumulation of sebum and the relative state of oxidation thereof in a standardised and objective manner.

Another object of the present invention is to provide a dependable, reliable and repeatable coding process.

These and other objects of the present invention are achieved by means of a process for coding an image of a scalp illuminated with a fluorescence-based Wood's light, characterised in that it comprises the steps of:
- extracting from the image a number of luminous spots per surface unit
- extracting from the image a dimension of the luminous spots
- extracting from the image a colour of the luminous spots
- extracting from the image a brightness of the luminous spots
- generating an alphanumeric code for coding the image made up of a first code field with a value derived from the colour of the luminous spots extracted from the image and the dimension of the luminous spots extracted from the image, a second code field with a value derived from the brightness of the luminous spots extracted from the image, and a third code field with a value derived from the number of luminous spots per surface unit extracted from the image.

The values of said first code field are associated, respectively, with an absence of colour, a yellowish/white colour, a yellow/red colour, an orange/red colour, and a red colour of the luminous spots.

The values of said first code field are further associated, respectively, with luminous spots that extend to the area of the follicular ostium and luminous spots that extend to the hair shaft.

The values of said second code field are associated, respectively, with dull luminous spots and with shiny luminous spots.

The values of said third code field are associated, respectively, with progressive numbers of luminous spots per surface unit.

Other salient aspects of the invention are disclosed in the dependent claims that follow.

Additional features and advantages of the invention will become more apparent from the description of a preferred but not exclusive embodiment of the process for coding an image of a scalp illuminated with a fluorescence-based Wood's light according to the present invention illustrated by way of non-limiting example in the appended drawing, in which:

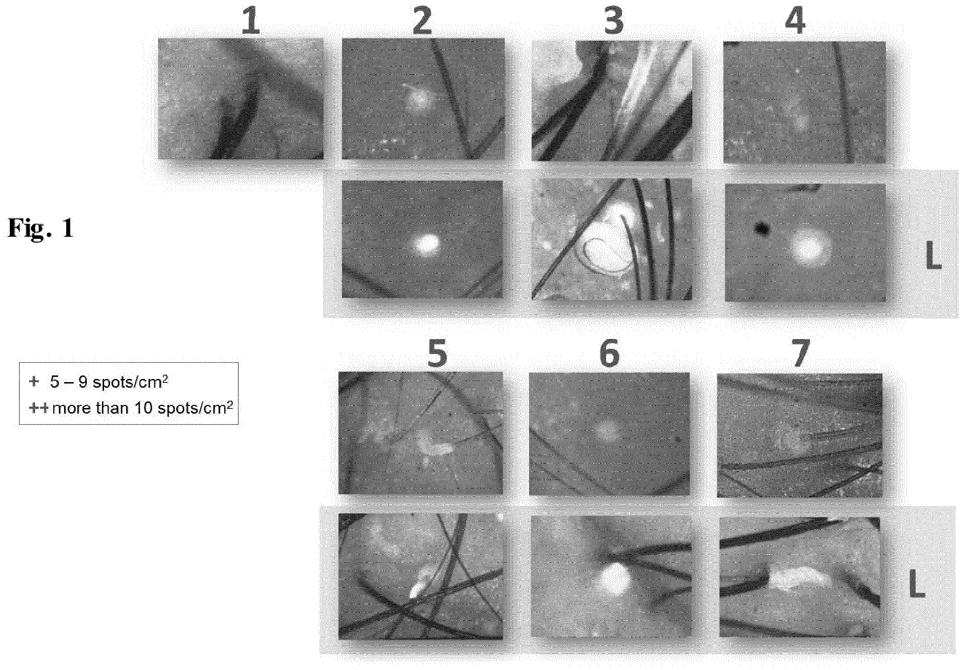

Figure 1 shows examples of codes of the reference scale created through the process of the present invention.

With reference to the aforesaid figure, it shows a process for coding an image of a scalp illuminated with a Wood's light.

The process, according to the present invention, comprises the steps of extracting from the image a number of luminous spots per surface unit, extracting from the image a dimension of the luminous spots, extracting from the image a colour of the luminous spots, and extracting from the image a brightness of the luminous spots.

The extraction of the aforesaid parameters can be performed by a human operator or automatically by analysing the image by means of an image analysis algorithm, possibly trained by a machine learning process or even a deep learning one that entails advanced automatic learning techniques based on **artificial neural networks.**

The process according to the present invention then comprises the steps of generating an alphanumeric code for coding the image made up of a first code field with a value associated with the colour of the luminous spots extracted from the image and with the dimension of the luminous spots extracted from the image, a second code field with a value associated with the brightness of the luminous spots extracted from the image, and a third code field with a value associated with the number of luminous spots per surface unit extracted from the image.

The values of said first code field are associated, respectively, with an absence of colour, a yellowish/white colour, a yellow/red colour, an orange/red colour, and a red colour of the luminous spots.

The values of said first code field are further associated, respectively, with luminous spots that extend to the area of the follicular ostium and with luminous spots that extend to the hair shaft. The values of said second code field are associated, respectively, with dull luminous spots and with shiny luminous spots.

The values of said third code field are associated, respectively, with progressive numbers of luminous spots per surface unit.

The values of the fields can also be calculated automatically by means of suitable calculation algorithms.

The image is preferably acquired by means of a 50x magnification optic.

A preferred embodiment of the process according to the present invention will be described in detail below.

### Materials and Methods

The means of trichological analysis used comprise a microcamera fitted with a 50x magnification optic, integrated Wood's light illuminator and polarising filter.

Images of the scalp and hair shaft are obtained, for example, by means of a microcamera from the company APR Instruments and associated software. The magnification used is 50x.

By means of a 50 FL magnification one obtains a photograph of the state of the scalp with particular reference to the accumulation of sebum. The 50 FL optic is a scalp analysis instrument that shows the accumulation of sebum and the oxidative state thereof. The accumulations of sebum appear with a fluorescence of varying intensity according to their degree of oxidation. The **50 FL optic is provided with an** integrated Wood's light illuminator, which, through a polarising filter, highlights the corneal sebaceous accumulations and the state of oxidation thereof, clarifying the chemical change of the sebum. Oxidised sebum has a greater irritant action and appears as a further cause of the acceleration of the ongoing degenerative processes.

### IMAGE CODING

The Wood's light objective with fluorescence, called 50FL, is able to highlight the corneal sebaceous accumulations, whose size is proportional to the extent of the detected signal and its brightness to the concentration of the accumulation.

The visible colour, on the other hand, indicates the different degree of oxidation: the greater the degree of oxidation, the greater its negative influence on surrounding tissues.

By way of example, codes with 7 values, in particular progressive numbers, of the first field were identified. The code can also comprise, in addition to the value of the first field, a value of the second and/or third field.

An empty field in the code can be used to identify the minimum value attributable to the field. Preferably, the code fields contain different categories of values, in particular at least one numerical digit in the first field, at least one letter in the second field and at least one algebraic sign in the third field.

### CLASSIFICATION PARAMETERS

The parameters used for coding the image are representative of the oxidative states of the sebum accumulated on the scalp of an individual's head, are precise, recognisable physical variables and are divided into: primary and secondary physical variables which determine the values of the first code field, tertiary physical variables which determine the values of the second code field, and quaternary physical variables which determine the values of the third code field.

The primary physical variable which determines the values of the first code field includes the colour of the luminous spots of the scalp illuminated with the fluorescence-based Wood's light. In particular, the colour of the luminous spots identifies a different state of oxidation, which increases in proportion to the negativity of the phenomenon.

The colour can be:
a) Yellowish/white
b) Yellow/red
c) Orange/red

The secondary physical variable which determines the values of the first code field includes the size of the luminous spots: the greater the extent (the spatial dimension identified), the larger the affected area, and therefore the negativity of the phenomenon is proportionally greater.

In particular, the luminous spots can be localised in the area of the follicular ostium or extend onto the hair shaft, above the area of the follicular ostium (one thus speaks of a sleeve-like protrusion).

The tertiary physical variable which determines the values of the second code field includes the intensity of the signal emitted by the luminous spots: the greater the intensity, the higher the concentration of the accumulation of sebum, and therefore the negativity of the phenomenon is proportionally greater.

The signal intensity of the luminous spots can appear dull or shiny.

The quaternary physical variable which determines the values of the third code field includes the number of luminous spots per surface unit: the more greatly affected the scalp is, the greater the negativity of the phenomenon will be.

The distribution of the luminous spots can be:
a) Low distribution: between 1 and 4 spots per cm²
b) Medium distribution: between 5 and 9 spots per cm²
c) High distribution: more than 10 spots per cm²

Codes with 7 progressive values of the first field most frequently identified are shown below by way of example.

The progressive order reflects the increasing degree of relevance of the state of oxidation of the sebum, i.e. one starts from a code with a first field with a value of 1, which identifies a situation of absence of luminous spots and thus of accumulations, eventually arriving at a code with first field with a value of 7, which represents the maximum degree of oxidation and extent of the sebaceous accumulation.

### CODE WITH A FIRST FIELD WITH A VALUE OF 1

As figure 1 shows, the code having a first field with a value of 1 identifies an image of a physiological skin with the absence of fluorescence.

### Primary physical variables:

Colour: none

### Secondary physical variables:

Size: zero

### Tertiary physical variables:

Signal intensity: none

### Quaternary physical variables:

Distribution: zero

Example of code with a first field with a value of 1: an image without fluorescence will be classified as with code 1, made up solely by the first field filled in with a value of 1.

### CODE WITH A FIRST FIELD WITH A VALUE OF 2

As figure 1 shows, the code having a first field with a value of 2 represents luminous spots localised in the area of the follicular ostium.

### Primary physical variables:

Colour: yellowish/white

### Secondary physical variables:

Size: localised in the area of the follicular ostium

### Tertiary physical variables:

### Signal intensity:

a)Low: dull signal
b)High: shiny signal (in the case of a shiny signal a value L of the second field is associated with the value of 2 of the first field)

### Quaternary physical variables:

### Distribution:

a)Between 1 and 4 spots per cm²
b)Between 5 and 9 spots per cm² (in the case of a distribution of between 5 and 9 spots per cm² a value + of the third field is associated with the value of 2 of the first field)
c)More than 10 spots per cm² (in the case of a distribution of more than 10 spots per cm² a value ++ of the third field is associated with the value of 2 of the first field)

Examples of code with a first field with a value of 2:
- an image with yellowish fluorescence, dull, flattened dimension at the follicular ostium with a high distribution (15 luminous spots per cm²) will be classified with the code 2++ having a first field with a value of 2 and a third field with a value ++.
- an image with yellowish fluorescence, shiny, flattened dimension at the follicular ostium with a medium distribution (8 luminous spots per cm²) will be classified with the code 2L+ having a first field with a value of 2, second field with a value L and third field with a value +.

### CODE WITH A FIRST FIELD WITH A VALUE OF 3

As figure 1 shows, the code with a first field with a value of 3 represents luminous spots showing yellowish/white fluorescence which protrude beyond the level of the follicular ostium.

### Primary physical variables:

Colour: yellowish/white

### Secondary physical variables:

Size: it extends onto the hair shaft, above the area of the follicular ostium

### Tertiary physical variables:

### Signal intensity:

a)Low: dull signal
b)High: shiny signal (in the case of a shiny signal a second field with a value L is associated with the first field with a value of 3)

### Quaternary physical variables:

### Distribution:

a)Between 1 and 4 spots per cm²
b)Between 5 and 9 spots per cm² (in the case of a distribution of between 5 and 9 spots per cm² a third field with a value + is associated with the first field with a value of 3)
c)More than 10 spots per cm² (in the case of a distribution of more than 10 spots per cm² a third field with a value ++ is associated with the first field with a value of 3)

Examples of code with a first field with a value of 3:
- an image with yellowish fluorescence, dull, accumulation extending onto the shaft beyond the follicular ostium with a high distribution (15 luminous spots per cm²) will be classified with the code 3++ having a first field with a value of 3 and third field with a value ++.
- an image with yellowish fluorescence, shiny, accumulation extending onto the shaft beyond the follicular ostium with a medium distribution (8 luminous spots per cm²) will be classified with the code 3L+ having a first field with a value of 3, second field with a value L and third field with a value +.

### CODE WITH A FIRST FIELD WITH A VALUE OF 4

As figure 1 shows, the code with a first field with a value of 4 represents luminous spots showing yellow/red fluorescence at the level of the follicular ostium.

### Primary physical variables:

Colour: yellow/red

### Secondary physical variables:

Size: localised in the area of the follicular ostium

### Tertiary physical variables:

### Signal intensity:

a)Low: dull signal
b)High: shiny signal (in the case of a shiny signal a second field with a value identified by the letter "L" is associated with the first field with a value of 4)

### Quaternary physical variables:

### Distribution:

a)Between 1 and 4 spots per cm²
b)Between 5 and 9 spots per cm² (in the case of a distribution of between 5 and 9 spots per cm² a third field with a value identified by the algebraic symbol "+" is associated with the first field with a value of 4)
c)More than 10 spots per cm² (in the case of a distribution of more than 10 spots per cm² a third field with a value identified by the algebraic symbol "++" is associated with the first field with a value of 4)

Examples of code with a first field with a value of 4:
- an image with yellow-red fluorescence, dull, localised on the follicular ostium with a high distribution (12 luminous spots per cm²) will be classified with the code 4++ having a first field with a value of 4 and third field with a value ++.
- an image with yellow-red fluorescence, shiny, localised on the follicular ostium with a low distribution (2 luminous spots per cm²) will be classified with the code 4L having a first field with a value of 4 and second field with a value L.

### CODE WITH A FIRST FIELD WITH A VALUE OF 5

As figure 1 shows, the code with a first field with a value of 5 represents luminous spots showing yellow/red fluorescence which protrudes above the level of the follicular ostium.

### Primary physical variables:

Colour: yellow/red

### Secondary physical variables:

Size: it extends onto the hair shaft, above the area of the follicular ostium;

### Tertiary physical variables:

### Signal intensity:

a)Low: dull signal
b)High: shiny signal (in the case of a shiny signal a second field with a value identified by the letter "L" is associated with the first field with a value of 5)

### Quaternary physical variables:

### Distribution:

a)Between 1 and 4 spots per cm²
b)Between 5 and 9 spots per cm² (in the case of a distribution of between 5 and 9 spots per cm² a third field with a value identified by the algebraic symbol "+"is associated with the first field with a value of 5)
c)More than 10 spots per cm² (in the case of a distribution of more than 10 spots per cm² a third field with a value identified by the algebraic symbol "++" is associated with the first field with a value of 5)

Examples of code with a first field with a value of 5:
- an image with yellow-red fluorescence, dull, accumulation extending onto the shaft beyond the follicular ostium with a high distribution (15 luminous spots per cm²) will be classified with the code 5++ having a first field with a value of 5 and third field with a value ++.
- an image with yellow-red fluorescence, shiny, accumulation extending onto the shaft beyond the follicular ostium with a medium distribution (8 luminous spots per cm²) will be classified with the code 5L+ having a first field with a value of 5, second field with a value L and third field with a value +.

### CODE WITH A FIRST FIELD WITH A VALUE OF 6

As figure 1 shows, the code with a first field with a value of 6 represents luminous spots showing localised orange/red fluorescence at the level of the follicular ostium.

### Primary physical variables:

Colour: orange/ red

### Secondary physical variables:

Size: localised in the area of the follicular ostium;

### Tertiary physical variables:

### Signal intensity:

a)Low: dull signal
b)High: shiny signal (in the case of a shiny signal a second field with a value identified by the letter "L" is associated with the first field with a value of 6)

### Quaternary physical variables:

### Distribution:

a)Between 1 and 4 spots per cm²
b)Between 5 and 9 spots per cm² (in the case of a distribution of between 5 and 9 spots per cm² a third field with a value identified by the algebraic symbol "+" is associated with the first field with a value of 6)
c)More than 10 spots per cm² (in the case of a distribution of more than 10 spots per cm² a third field with a value identified by the algebraic symbol "++" is associated with the first field with a value of 6)

Examples of code with a first field with a value of 6:
- an image with orange-red fluorescence, dull, localised at the level of the follicular ostium with a medium distribution (7 luminous spots per cm²) will be classified with the code 6+ having a first field with a value of 6 and third field with a value + .
- an image with orange-red fluorescence, shiny, localised at the level of the follicular ostium with a high distribution (18 luminous spots per cm²) will be classified with the code 6L++ having a first field with a value of 6, second field with a value L and third field with a value ++ .

### CODE WITH A FIRST FIELD WITH A VALUE OF 7

As figure 1 shows, the code with a first field with a value of 7 represents luminous spots showing red fluorescence protruding along the shaft, above the follicular ostium.

### Primary physical variables:

Colour: red

### Secondary physical variables:

Size: it extends onto the shaft, above the area of the follicular ostium;

### Tertiary physical variables:

### Signal intensity:

a)Low: dull signal
b)High: shiny signal (in the case of a shiny signal a second field with a value identified by the letter "L" is associated with the first field with a value of 7)

### Quaternary physical variables:

### Distribution:

a)Between 1 and 4 spots per cm²
b)Between 5 and 9 spots per cm² (in the case of a distribution of between 5 and 9 spots per cm² a third field with a value identified by the algebraic symbol "+" is associated with the first field with a value of 7)
c)More than 10 spots per cm² (in the case of a distribution of more than 10 spots per cm² a third field with a value identified by the algebraic symbol "++" is associated with the first field with a value of 7)

Examples of code with a first field with a value of 7:
- an image with red fluorescence, dull, accumulation extending onto the shaft beyond the follicular ostium with a low distribution (4 luminous spots per cm²) will be classified with the code 7 having a first field with a value of 7 .
- an image with fluorescence red, shiny, accumulation extending onto the shaft beyond the follicular ostium with a medium distribution (5 luminous spots per cm²) will be classified with a code having a first field with a value of 7, second field with a value L and third field with a value +.

The image coding representative of the states of oxidation of the sebum accumulated on the scalp produced by means of the process of the present invention makes it possible to standardise the analytic method and build reusable databases.

The process according to the present invention has enabled the purely qualitative data deriving from images of the scalp used up to today in trichological analysis to be transformed into quantitative data.

The process thus conceived is susceptible of numerous modifications and variants, all falling within the scope of the inventive concept described and claimed.

## Claims

1. A process for coding an image of a scalp illuminated with a fluorescence-based Wood's light, **characterised in that** it comprises the steps of:
- extracting from the image a number of luminous spots per surface unit
- extracting from the image a dimension of the luminous spots
- extracting from the image a colour of the luminous spots
- extracting from the image a brightness of the luminous spots
- generating an alphanumeric code for coding the image made up of a first code field containing a value derived from the colour of the luminous spots extracted from the image and from the dimension of the luminous spots extracted from the image, a second code field containing a value derived from the brightness of the luminous spots extracted from the image, and a third code field containing a value derived from the number of luminous spots per surface unit extracted from the image.

2. The process according to claim 1, **characterised in that** said image is acquired by means of a 50× magnification optic.

3. The process according to any preceding claim, **characterised in that** the values of said first field are associated, respectively, with an absence of colour, a yellowish/white colour, a yellow/red colour, an orange/red colour, and a red colour of the luminous spots.

4. The process according to the preceding claim, **characterised in that** the values of said first field are further associated, respectively, with luminous spots that extend to the area of the follicular ostium and with luminous spots that extend to the hair shaft.

5. The process according to any preceding claim, **characterised in that** the values of said second field are associated, respectively, with dull luminous spots and with shiny luminous spots.

6. The process according to any preceding claim, **characterised in that** the values of said third field are associated, respectively, with progressive numbers of luminous spots per surface unit.

7. The process according to any preceding claim, **characterised in that** the values of said first field include at least one numerical digit.

8. The process according to any preceding claim, **characterised in that** the values of said second field include at least one letter.

9. The process according to any preceding claim, **characterised in that** the values of said third field include at least one algebraic sign.

10. A coding scale of states of oxidation of the sebum accumulated on the scalp obtained with a process for coding images of a scalp illuminated with a Wood's light according to any preceding claim.
